# EUROPÄISCHE PATENTSCHRIFT

(11) **EP 1 041 069 B1**
(45) Veröffentlichungstag und Bekanntmachung des Hinweises auf die Patenterteilung: **06.02.2002**
(21) Anmeldenummer: 00104399.1
(22) Anmeldetag: 02.03.2000
(51) Int. Cl.: C07D 251/28

(54) **Rieselfähiges Cyanurchlorid, Verfahren zu seiner Herstellung und dessen Verwendung**
Free-flowing cyanuric chloride, process for its preparation and its use
Chlorure de cyanuryle s'écoulant librement, procédé de sa préparation et son usage

(30) Priorität: 31.03.1999 DE 19914616
(43) Veröffentlichungstag der Anmeldung: 04.10.2000
(73) Patentinhaber: Degussa AG, 40474 Düsseldorf (DE)
(72) Erfinder: Schmidt, Manfred, Dr., 63571 Gelnhausen (DE); Leutner, Josef, 63920 Grossheubach (DE)

(56) Entgegenhaltungen:
- EP-A- 0 416 584
- DE-B- 1 134 999
- DE-B- 2 839 384

## Beschreibung

Die Erfindung richtet auf rieselfähiges Cyanurchlorid, das als Rieselhilfsmittel eine hydrophile Kieselsäure enthält, mit gegenüber vorbekanntem, eine pyrogene Kieselsäure enthaltendem Cyanurchlorid verbessertem Eigenschaftsprofil. Die Erfindung richtet sich ferner auf ein Verfahren zur Herstellung des verbesserten rieselfähigen Cyanurchlorids sowie dessen Verwendung.

Es ist bekannt, daß festes Cyanurchlorid in feinteiliger Form leicht zusammenbackt. Um die dadurch bedingten Probleme bei der Lagerung und Weiterverarbeitung zu beheben, werden gemäß DE-Auslegeschrift 11 34 999 dem feinteiligen Cyanurchlorid Rieselhilfsmittel aus der Reihe von fein verteiltem Siliciumdioxid, Titandioxid, Aluminiumoxid, Aluminiumsilicat und Calciumsilicat in einer Menge von 0,3 bis 3 Gew.-% zugefügt. Unter der in diesem Dokument genannten Kieselsäure als Rieselhilfsmittel wird ausschließlich eine pyrogen erzeugte Kieselsäure, nämlich Aerosil®, genannt. Anregungen, Kieselsäuren anderer Provenienz zu verwenden, werden nicht gegeben.

Wie aus der DE-Auslegeschrift 28 39 384 hervorgeht, ist es durch Verwendung einer hydrophoben pyrogenen oder hydrophoben gefällten Kieselsäure möglich, nicht nur die Rieselfähigkeit von Cyanurchlorid zu verbessern, sondern - auch der bei der Lagerung und innerbetrieblichen Handhabung von Cyanurchlorid auftretenden unerwünschten Hydrolyse entgegenzuwirken. Während der Lagerung und Umsetzung von Cyanurchlorid gebildete Hydrolyseprodukte mindern die Qualität und/oder die Ausbeute der Umsetzungsprodukte.

Im Verfahren gemäß EP-A 0 416 584 wurde versucht, die Fließfähigkeit von festem Cyanurchlorid dadurch zu verbessern, daß Cyanurchlorid unter Erwärmen auf eine Temperatur unterhalb des Schmelzpunktes in einem Kneter oder Mischer einer Scherbeanspruchung unterworfen wird. Es wurde aber festgestellt - siehe Vergleichsbeispiele - daß die Reaktivität derartiger Produkte gemindert ist. Wegen ungenügender Lagerstabilität so behandelter Produkte, konnte sich dieses Verfahren in der Praxis nicht durchsetzen, so daß die meisten Handelsprodukte als Rieselhilfsmittel weiterhin eine hydrophile pyrogene Kieselsäure oder eine hydrophobe pyrogene oder gefällte Kieselsäure enthalten.

Ein wesentlicher Nachteil aller hydrophilen als auch hydrophoben pyrogenen Kieselsäuren sowie auch der hydrophoben gefällten Kieselsäuren ist deren herstellungsbedingtes hohes Preisniveau. Die Fachwelt ist daher an preisgünstigeren Alternativen interessiert, wobei diese Alternativen zumindest ein gleichwertiges Eigenschaftsprofil aufweisen müssen.

Aufgabe der vorliegenden Erfindung ist demgemäß, die Bereitstellung eines wirtschaftlicher zugänglichen rieselfähigen Cyanurchlorids mit einem Eigenschaftsprofil, das jenem der derzeitigen Handelsprodukte zumindest gleichkommt, vorzugsweise dieses aber in dem einen oder anderen Punkt übertrifft. Bei dem Eigenschaftsprofil handelt es sich im wesentlichen um
(i) eine möglichst hohe Schüttdichte
(ii) eine gute Rieselfähigkeit entsprechend einer Bewertungszahl von gleich oder kleiner 3, vorzugsweise gleich oder kleiner 2, und
(iii) eine möglichst hohe Reaktivität, wobei letztere gegenüber unbehandeltem Cyanurchlorid möglichst nicht verschlechtert, vorzugsweise sogar verbessert sein sollte.

Die Aufgabe wird gelöst durch ein rieselfähiges Cyanurchlorid mit einer Rieselfähigkeits-Bewertungszahl von gleich oder kleiner 3, enthaltend als Rieselhilfsmittel eine hydrophile Kieselsäure, welches dadurch gekennzeichnet ist, daß die hydrophile Kieselsäure eine Fällungskieselsäure oder ein Kieselgel mit einem mittleren Agglomeratdurchmesser von kleiner 15 µm, bestimmt mit dem Coulter Counter, ist. Die Unteransprüche richten sich auf bevorzugte Ausführungsformen des rieselfähigen Cyanurchlorids. Bevorzugt werden Fällungskieselsäuren.

Bevorzugte Produkte weisen einen mittleren Agglomeratdurchmesser von kleiner 7 um, vorzugsweise kleiner 5 µm auf. Es wurde ferner festgestellt, daß insbesondere hydrophile Fällungskieselsäuren mit einer DBP-Absorptiön von unterhalb 270 g/100 g ein besonders gutes Eigenschaftsprofil des rieselfähigen Cyanurchlorids abgeben. Herstellungsbedingt weisen Fällungskieselsäuren und Kieselgele unterschiedliche Strukturen auf. Vorzugsweise handelt es sich bei den Rieselhilfsmitteln um gemahlene Fällungskieselsäuren, insbesondere dampf- oder luftstrahlgemahlene Fällungskieselsäuren mit einem Agglomeratdurchmesser von kleiner 5 um und einer DBP-Absorption im Bereich von 200 bis 260 g/100 g. Bei den in Frage kommenden Fällungskieselsäuren und Kieselgelen handelt es sich üblicherweise um Produkte mit einer BET-Oberfläche von mindestens 100 m²/g; vorzugsweise werden Produkte mit einer BET-Oberfläche im Bereich von 100 bis 500 m²/g verwendet, besonders bevorzugt 150 bis 300 m²/g.

Ein besonders wichtiges Kriterium für das Eigenschaftsprofil des rieselfähigen Cyanurchlorids ist die unter Einsatz des Rieselhilfsmittels und unter definierten Mischbedingungen erzielte Schüttdichte. Im Hinblick auf das Verpackungsvolumen des rieselfähigen Cyanurchlorids ist es von besonderem Interesse, eine möglichst hohe Schüttdichte zu erzielen. Das absolute Schüttgewicht hängt nicht nur vom Rieselhilfsmittelgehalt und den Mischbedingungen ab, sondern richtet sich verständlicherweise auch nach der Kornverteilung des eingesetzten Cyanurchlorids. Es wurde gefunden, daß unter Verwendung erfindungsgemäß einzusetzender Rieselhilfsmittel eine höhere Schüttdichte erzielbar ist als unter Verwendung einer hydrophilen pyrogenen Kieselsäure, wie Aerosil® 200. Die Schüttdichte eines bevorzugten rieselfähigen Cyanurchlorids ist vergleichbar mit jener, welche unter Verwendung einer hydrophoben gefällten Kieselsäure erzielt wird, jedoch überraschenderweise höher als jene, die unter Einsatz einer hydrophilen pyrogenen Kieselsäure bei gleicher Einsatzmenge und gleichen Mischbedingungen erhältlich ist.

Ein weiteres Kriterium des Eigenschaftsprofils ist die Rieselfähigkeit: Die Bewertungszahlen resultieren aus den Ergebnissen unter Verwendung standardisierter Auslauftrichter mit Auslaufweiten im Bereich zwischen 2,5 und 18 mm. Ein Produkt, das aus einem Trichter mit einer Öffnungsweite von 8 beziehungsweise 5 mm stockungsfrei ausläuft, erhält eine Bewertungszahl von 3 beziehungsweise 2. Produkte mit einer Bewertungszahl von 2 oder kleiner 2 werden bevorzugt. Fällungskieselsäuren und Kieselgele mit einem Agglomeratdurchmesser von über 15 µm führen zu keiner befriedigenden Rieselfähigkeits-Bewertungszahl.

Die Reaktivität des rieselfähigen Cyanurchlorids stellt das dritte Kriterium des geforderten Eigenschaftsprofils dar. Die Bestimmung der Reaktivität basiert darauf, daß Cyanurchlorid in wäßrigem Medium unter definierten Bedingungen hydrolysiert und dabei die Zeit bis zum Erreichen eines pH-Wertes von 7 gemessen wird. Angestrebt wird eine möglichst hohe Reaktivität, d. h. eine geringe Zeit zum Erreichen des pH-Wertes von 7. Während durch die Verwendung einer hydrophoben gefällten oder pyrogenen Kieselsäure die Reaktivität gegenüber unbehandeltem Cyanurchlorid deutlich abnimmt, entspricht die Reaktivität von erfindungsgemäßem rieselfähigen Cyanurchlorid im wesentlichen unbehandeltem Cyanurchlorid, und vorzugsweise übersteigt die Reaktivität erfindungsgemäßer Produkte jene des unbehandelten Cyanurchlorids. Die Reaktivität erfindungsgemäßer Produkte liegt im Bereich jener, welche unter Einsatz einer hydrophilen pyrogenen Kieselsäure erhältlich ist; bevorzugte Produkte weisen jedoch eine noch höhere Reaktivität auf.

Ein unerwarteter Vorteil erfindungsgemäßer Produkte liegt auch darin, daß diese Produkte einen außergewöhnlich niedrigen Hydrolysatgehalt aufweisen. Bei dem Hydrolysatgehalt handelt es sich um in Toluol unlösliche Produkte, welche durch Hydrolyse des Cyanurchlorids entstanden sind. Der Hydrolysatgehalt wird hierbei nach einer bestimmten Lagerzeit nach Herstellung des rieselfähigen Cyanurchlorids ermittelt. Es wurde gefunden, daß unter Einsatz der bevorzugten Fällungskieselsäuren und Kieselgele der Hydrolysatgehalt überraschenderweise wesentlich niedriger ist als jener, welcher unter Einsatz hydrophober Kieselsäuren erhalten wird. Dies ist deswegen überraschend, weil bisher hydrophobe Kieselsäuren speziell verwendet wurden, um die hydrolyseempfindlichen Cyanurchloridpartikel wirksam zu umhüllen und damit Hydrolyse zu erschweren.

Bei den erfindungsgemäß zu verwendenden Fällungskieselsäuren oder Kieselgelen handelt es sich um Produkte, welche herstellungsbedingt preisgünstiger erhältlich sind als pyrogen erzeugte oder/und hydrophobe Kieselsäuren. Weitere Vorteile sind die hohe Reaktivität und die hohe Schüttdichte der Produkte. Ein zusätzlicher Vorteil erfindungsgemäßer Produkte besteht darin, daß es beim Entleeren von Verpackungen zu keiner statischen Aufladung des Produkts und somit zu keinem Anhaften desselben am Verpackungsmaterial kommt.

Die Einsatzmenge an Rieselhilfsmittel liegt im Bereich von 0,05 bis 5 Gew.-%, vorzugsweise 0,05 bis 0,5 Gew.-% und insbesondere 0,1 bis 0,3 Gew.-%.

Die Herstellung der erfindungsgemäßen Produkte erfolgt in an sich bekannter Weise durch homogenes Mischen des Cyanurchloridpulvers mit dem Rieselhilfsmittel. Hierbei werden übliche Mischeinrichtungen verwendet. Zwar kann durch Verlängerung der Mischdauer das Schüttgewicht erhöht werden, jedoch bleibt der zuvor dargestellte Unterschied zwischen den erfindungsgemäß zu verwendenden hydrophilen Kieselsäuren und einer pyrogenen hydrophilen Kieselsäure bestehen. Das Zumischen des Rieselhilfsmittels kann unmittelbar nach der Herstellung des pulverförmigen Cyanurchlorids erfolgen oder durch einen separaten Mischungsprozeß bewerkstelligt werden.

Durch die Aufrechterhaltung einer hohen Reaktivität des rieselfähigen Cyanurchlorids läßt sich dieses zur Herstellung von Cyanurchloridderivaten, bei denen ein, zwei oder drei Chloratome des Cyanurchlorids substituiert sind, verwenden. Die hohe Reaktivität und der geringe Hydrolysatgehalt führen hierbei zu kurzen Reaktionszeiten und reinen Produkten.

Die Erfindung wird anhand der nachfolgenden Beispiele und Vergleichsbeispiele näher erläutert; dargestellt werden auch die Testmethoden.

### Bestimmung der Schüttdichte (SD)

Ein 100 ml Meßzylinder - exakt bei 100 ml abgeschnitten - wird mittels eines Trichters mit Produkt aufgefüllt und der überstehende Schüttkegel abgestreift. Der Meßzylinder wird von äußerlich anhaftendem Produkt gereinigt und anschließdend ausgewogen. Angabe in g/l.

### Bestimmung der Rieselfähigkeit (RF)

Die zu prüfende Probe wird in ein zylinderförmiges, im unteren Teil trichterförmig ausgebildetes Auslaufgefäß gegeben, dessen Auslauföffnung dabei verschlossen gehalten wird. Öffnet man die Auslauföffnung, rieselt das Produkt stockungsfrei aus. Um den Auslaufvorgang zu starten, kann im Bedarfsfall mit einem Spatel gegen das Gefäß geklopft werden. Es stehen 5 Auslaufgefäße mit unterschiedlich großen Auslauföffnungen zur Verfügung.

### Gefäße: Höhe = 90 mm, Innen Ø = 42 mm

| Gefäß (Nr.) | Auslauföffung Ø (mm) |
|---|---|
| 1 | 2,5 |
| 2 | 5 |
| 3 | 8 |
| 4 | 12 |
| 5 | 18 |

Bewertungszahl: 1 = läuft glatt durch Gefäß 1 (kleinste Öffnung)
5 = läuft durch Gefäß 5 (größte Öffnung)
6 = läuft nicht durch Gefäß 5

### Bestimmung der Reaktivität (RK)

In wäßriger Lösung unterliegt Cyanurchlorid einer hydrolytischen Zersetzung. Cyanurchlorid hydrolisiert zu Cyanursäure. Von dieser mit fallendem pH-Wert, je nach der Reaktivität mehr oder weniger schnell verlaufenden Reaktion macht man Gebrauch bei der Bestimmung der Reaktivität von Cyanurchlorid. Mittels pH-Meter läßt sich die Abnahme des pH-Wertes verfolgen. Als Maßzahl der Reaktivität wird die Zeit (Min.) bis zum Erreichen von pH 7 verwendet.

Genau 9,22 g Cyanurchlorid (0,05 Mol) der Probe werden in ein 150 ml-Becherglas eingewogen, 1 Rührfisch zugegeben und auf ein Magnetrührwerk gestellt. Es werden genau 100 ml 1N NaOH (0,1 Mol) hinzupipettiert. Bei Zugabe der NaOH wird sofort die Stoppuhr gestartet, außerdem das Rührwerk angestellt und so stark gerührt, daß gute Durchmischung erfolgt. Nach genau 1 Min. wird das Becherglas in das 30 °C temperierte Wasserbad gestellt und die pH-Elektrode eingetaucht. Unter starkem Rühren (das Cyanurchlorid muß in der Lösung verwirbeln) wird die NaOH-Abnahme verfolgt. Bei Erreichen von pH 7 ist der Endpunkt der Bestimmung erreicht - die Zeit wird gestoppt.

### Bestimmung des Hydrolysatgehalts

Auf einer Präzisionswaage werden etwa 5 g zu untersuchendes Cyanurchlorid auf 0,1 g genau in einen Schlifferlenmeyer eingewogen und mit ca. 100 ml Toluol versetzt. Nach Verschließen des Erlenmeyers wird der Auflösungsprozeß des Cyanurchlorids durch Schütteln beschleunigt. Läßt sich nach vollständigem Auflösen des Cyanurchlorids visuell keine Trübung feststellen, so ist der Hydrolysatgehalt < 0,1 %, die Bestimmung kann abgebrochen werden. Bei Trübung oder Bildung eines Niederschlages filtriert man die Lösung durch einen getrockneten und tarierten Glasfiltertiegel und wäscht mit 20 ml Toluol nach. Der Filtertiegel ist im Trockenschrank bei etwa 120 °C bis zur Gewichtskonstanz zu trocknen. Die Auswaage entspricht der Summe aus Hydrolysat und Rieselhilfsmittel. Durch Auswaschen des Niederschlages mit heißem Wasser und Trocknen des Rückstands läßt sich der Anteil Rieselhilfsmittel ermitteln.

### Beispiele B1 bis B3 und Vergleichsbeispiele VB1 bis VB3

Hergestellt wurde ein rieselfähiges Cyanurchlorid durch 15-minütiges Mischen von feinteiligem Cyanurchlorid (Charge 391) mit 0,3 Gew.-% der angegebenen Kieselsäure (KS) in einem Behälter auf einem Rollbock, wobei der Behälter langsam rollte. Aus der Tabelle 1 folgen die Ergebnisse für das Schüttgewicht (SD), die Reaktivität (RK) und die Rieselfähigkeit (RF).

**Tabelle 1**

| **Nr.** | **KS** **∗)** **(Typ)** | **SD (g/l)** | **RK (Min.)** | **RF** |
|---|---|---|---|---|
| **VB1** | - | 655 | 7,5 | 4 - 5 |
| **VB2** | Aerosil® 200 | 746 | 8,5 | 2 |
| | hydrophil, pyrogen | | | |
| **VB3** | Aerosil® R812 | 764 | 18,5 | 2 |
| | hydrophob, pyrogen | | | |
| **B1** | FK 320 DS | 778 | 6,7 | 1 |
| | hydrophil, gefällt; | | | |
| | d₅₀ = 4 µm; | | | |
| | DBP-Absorption 230g/100g | | | |
| **B2** | Sipernat 50 S | 790 | 7,5 | 3 - 4 ∗∗) |
| | hydrophil, gefällt, | | | |
| | DBP- Absorption 330g/100g | | | |
| **B3** | FK 500 LS | 755 | 6,8 | 3 ∗∗) |
| | hydrophil, gefällt; | | | |
| | d₅₀ = 3,5 µm; | | | |
| | DBP-Absorption 330g/100g | | | |

| | | | | |
|---|---|---|---|---|
| ∗) Bei den eingesetzten Produkten handelt es sich um Kieselsäuren der Degussa-Hüls AG. Bei den Kieselsäuren der Beispiele B1 bis B3 handelt es sich um gemahlene Produkte. | | | | |
| ∗∗)Mit einem etwas höheren Gehalt an Rieselhilsmittel oder eine Abmischung von Sipernat 50 S bzw. FK 500 LS mit beispielsweise FK 320 DS werden RF-Werte von unter 3 erreicht. | | | | |

### Beispiel B4 und B5 und Vergleichsbeispiele VB4 bis VB6

Cyanurchlorid (CC)(Charge 391) sowie eine noch feinteiligere Charge (Charge 399) wurden unter Einsatz von 0,25 Gew.-% Aerosol® beziehungsweise FK 320 DS rieselfähig gemacht. Die Tabelle zeigt die Ergebnisse. Mittelwerte aus 5 Bestimmungen.

**Tabelle 2**

| **Nr.** | **CC∗ (Typ)** | **KS (Typ)** | **SD (g/l)** | **RK (Min)** | **RF** |
|---|---|---|---|---|---|
| **VB1** | 391 | - | 655 | 7,5 | 4 - 5 |
| **VB4** | 391 | Aerosil® 200 | 733 | 7,9 | 2 |
| **B4** | 391 | FK 320 DS | 773 | 7,0 | 2 |
| **VB5** | 399 | - | 636 | 7,6 | 4 - 5 |
| **VB6** | 399 | Aerosil® 200 | 795 | 6,8 | 2 |
| **B5** | 399 | FK 320 DS | 830 | 6,8 | 1 - 2 |

### Beispiel B6 und Vergleichsbeispiele VB7 bis VB8

Hergestellt wurde rieselfähiges Cyanurchlorid, wobei das eingesetzte Cyanurchlorid weniger feinteilig war als die Chargen 391 und 399. Der Gehalt an Rieselhilfsmittel betrug 0,3 Gew.-%. Eingemischt wurde 15 Minuten im Mischfaß, das auf einem Rollbock rotierte. Die Ergebnisse folgen aus der Tabelle 3. Angegeben sind die Mittelwerte der Schüttdichte, der Reaktivität sowie des Hydrolysatgehalts und RF-Werts aus je 10 Bestimmungen sowie die Standardabweichung s in %.

**Tabelle 3**

| **Nr.** | **KS (Typ)** | **SD (g/l) (s %)** | **RK (Min.) (s %)** | **RF** | **Hydrolysat (%) (s %)** |
|---|---|---|---|---|---|
| VB7 | - | 731 | 12,1 | 4 - 5 | < 0,05 |
| B4 | FK 320 DS | 866 (0,7) | 10,9 (8) | 1 | 0,33 (1,6) |
| | gefällt; | | | | |
| | hydrophil | | | | |
| VB8 | Aerosil® | 844 (0,6) | 14,8 (7,2) | 1 - 2 | 0,47 (4,8) |
| | R 974 | | | | |
| | hydrophob | | | | |

Die Beispiele zeigen die herausragende Eigenschaftskombination im Vergleich zu jener der Vergleichsbeispiele.

## Patentansprüche

1. Rieselfähiges Cyanurchlorid mit einer Rieselfähigkeits-Bewertungszahl von gleich oder kleiner 3, enthaltend als Rieselhilfsmittel eine hydrophile Kieselsäure,
**dadurch gekennzeichnet,**
**daß** die hydrophile Kieselsäure eine Fällungskieselsäure oder ein Kieselgel mit einem mittleren Agglomeratdurchmesser von kleiner 15 µm, bestimmt mit dem Coulter Counter, ist.

2. Rieselfähiges Cyanurchlorid nach Anspruch 1,
**dadurch gekennzeichnet,**
**daß** die hydrophile Kieselsäure eine DBP-Absorption von kleiner 270 g/100 g aufweist.

3. Rieselfähiges Cyanurchlorid nach Anspruch 1 oder 2,
**dadurch gekennzeichnet,**
**daß** die hydrophile Kieselsäure gemahlen ist und einen mittleren Agglomeratsdurchmesser von kleiner 7 µm aufweist.

4. Rieselfähiges Cyanurchlorid nach einem der Ansprüche 1 bis 3,
**dadurch gekennzeichnet,**
**daß** das Rieselhilfsmittel eine dampf- oder luftstrahlgemahlene Fällungskieselsäure mit einem mittleren Agglomeratdurchmesser von gleich oder kleiner 5 µm ist.

5. Rieselfähiges Cyanurchlorid nach einem der Anprüche 2 bis 4,
**dadurch gekennzeichnet,**
**daß** die hydrophile Kieselsäure eine Fällungskieselsäure ist und eine DBP-Absorptionszahl nach DIN 53601 von unter 270 g/100 g aufweist.

6. Rieselfähiges Cyanurchlorid nach einem der Ansprüche 1 bis 5,
**dadurch gekennzeichnet,**
**daß** die Fällungskieselsäure eine BET-Oberfläche von 100 bis 500 m²/g, insbesondere 150 bis 300 m²/g, aufweist.

7. Rieselfähiges Cyanurchlorid nach einem der Ansprüche 1 bis 6,
**dadurch gekennzeichnet,**
**daß** es mindestens eine oder mehrere gefällte Kieselsäuren oder/und Kieselgele mit einem mittleren Agglomeratdurchmesser von gleich oder kleiner 15 µm in einer Gesamtmenge von 0,05 bis 0,5 Gew.-% enthält.

8. Verfahren zur Herstellung eines rieselfähigen Cyanurchlorids gemäß einem der Ansprüche 1 bis 6,
**dadurch gekennzeichnet,**
**daß** man das Cyanurchlorid und eine oder mehrere gefällte Kieselsäuren oder/und Kieselgele mit einem Agglomeratdurchmesser von kleiner 15 µm homogen miteinander mischt.

9. Verwendung eines rieselfähigen Cyanurchlorids gemäß einem der Ansprüche 1 bis 7 zur Herstellung von Cyanurchloridderivaten, bei denen ein, zwei oder drei Chloratome des Cyanurchlorids substituiert sind.

## Claims

1. Flowable cyanuric chloride having a flowability rating of less than or equal to 3 containing a hydrophilic silica as flow auxiliary,
**characterised in that**
the hydrophilic silica is a precipitated silica or silica gel having an average agglomerate diameter of less than 15 µm, determined using a Coulter Counter.

2. Flowable cyanuric chloride according to claim 1,
**characterised in that**
the hydrophilic silica exhibits a DBP absorption of less than 270 g/100 g.

3. Flowable cyanuric chloride according to claim 1 or 2,
**characterised in that**
the hydrophilic silica is ground and exhibits an average agglomerate diameter of less than 7 µm.

4. Flowable cyanuric chloride according to one of claims 1 to 3,
**characterised in that**
the flow auxiliary is a steam or air jet ground precipitated silica having an average agglomerate diameter of less than or equal to 5 µm.

5. Flowable cyanuric chloride according to one of claims 2 to 4,
**characterised in that**
the hydrophilic silica is a precipitated silica and exhibits a DBP absorption value to DIN 53601 of below 270 g/100 g.

6. Flowable cyanuric chloride according to one of claims 1 to 5,
**characterised in that**
the precipitated silica exhibits a BET surface area of 100 to 500 m²/g, in particular of 150 to 300 m²/g.

7. Flowable cyanuric chloride according to one of claims 1 to 6,
**characterised in that**
it contains at least one or more precipitated silicas and/or silica gels having an average agglomerate diameter of less than or equal to 15 µm in a total quantity of 0.05 to 0.5 wt.%.

8. Process for the production of a flowable cyanuric chloride according to one of claims 1 to 6,
**characterised in that**
the cyanuric chloride and one or more precipitated silicas or/and silica gels having an agglomerate diameter of less than 15 µm are homogeneously mixed together.

9. Use of a flowable cyanuric chloride according to one of claims 1 to 7 for the production of cyanuric chloride derivatives in which one, two or three chlorine atoms of the cyanuric chloride are substituted.

## Revendications

1. Chlorure de cyanuryle s'écoulant librement, ayant un indice d'évaluation de l'aptitude à l'écoulement égal ou inférieur à 3, contenant une silice hydrophile en tant qu'agent facilitant l'écoulement,
**caractérisé en ce que**
la silice hydrophile est une silice précipitée ou un gel de silice ayant un diamètre moyen d'agglomérat inférieur à 15 µm, déterminé à l'aide du compteur de particules Coulter Counter.

2. Chlorure de cyanuryle s'écoulant librement selon la revendication 1,
**caractérisé en ce que**
la silice hydrophile présente une absorption de DBP inférieure à 270 g/100 g.

3. Chlorure de cyanuryle s'écoulant librement selon la revendication 1 ou 2,
**caractérisé en ce que**
la silice hydrophile est broyée et présente un diamètre moyen d'agglomérat inférieur à 7 µm.

4. Chlorure de cyanuryle s'écoulant librement selon l'une quelconque des revendication 1 à 3,
**caractérisé en ce que**
l'agent facilitant l'écoulement est une silice précipitée, broyée au jet d'air ou de vapeur, ayant un diamètre moyen d'agglomérat ≤ 5 µm.

5. Chlorure de cyanuryle s'écoulant librement selon l'une quelconque des revendication 1 à 4,
**caractérisé en ce que**
la silice hydrophile est une silice précipitée et présente un indice d'absorption de DBP, selon DIN 53601, inférieur à 270 g/100 g.

6. Chlorure de cyanuryle s'écoulant librement selon l'une quelconque des revendication 1 à 5,
**caractérisé en ce que**
la silice précipitée présente une surface BET de 100 à 500 m²/g, en particulier de 150 à 300 m²/g.

7. Chlorure de cyanuryle s'écoulant librement selon l'une quelconque des revendication 1 à 6,
**caractérisé en ce qu'**
il contient une quantité totale de 0,05 à 0,5 % en poids d'une ou plusieurs silices précipitées et/ou gels de silice ayant un diamètre moyen d'agglomérat égal ou inférieur à 15 µm.

8. Procédé pour la préparation d'un chlorure de cyanuryle s'écoulant librement selon l'une quelconque des revendications 1 à 6,
**caractérisé en ce qu'**
on mélange entre eux de façon homogène le chlorure de cyanuryle et une ou plusieurs silices précipitées et/ou gels de silice ayant un diamètre d'agglomérat inférieur à 15 µm.

9. Utilisation d'un chlorure de cyanuryle s'écoulant librement selon l'une quelconque des revendications 1 à 7, pour la préparation de dérivés de chlorure de cyanuryle, dans lesquels 1, 2 ou 3 atomes de chlore du chlorure de cyanuryle sont remplacés.
